# EUROPÄISCHE PATENTSCHRIFT

(11) **EP 1 284 690 B1**
(45) Veröffentlichungstag und Bekanntmachung des Hinweises auf die Patenterteilung: **05.12.2007**
(21) Anmeldenummer: 01936383.7
(22) Anmeldetag: 21.05.2001
(51) Int. Cl.: A61F 2/46, A61B 17/16

(54) **INSTRUMENTARIUM ZUM EINSETZEN EINER HÜFTPFANNENPROTHESE**
INSTRUMENTS USED FOR INSERTING A HIP CUP
INSTRUMENTS POUR METTRE EN PLACE UNE PROTHESE COTYLOIDIENNE

(30) Priorität: 26.05.2000 DE 10026157
(43) Veröffentlichungstag der Anmeldung: 26.02.2003
(73) Patentinhaber: WALDEMAR LINK GmbH & Co. KG, 22339 Hamburg (DE)
(72) Erfinder: CHUNG, Wui K., Penrith NSW 2750 (AU); RIZKALLAH, Sherif M., Dubbo, NSW 2830 (AU); KELLER, Arnold, 23863 Kayhude (DE)
(74) Vertreter: Glawe, Delfs, Moll
(86) Internationale Anmeldenummer: PCT/EP2001/005821
(87) Internationale Veröffentlichungsnummer: WO 2001/091673

(56) Entgegenhaltungen:
- EP-A- 0 583 975
- EP-A- 0 687 452
- EP-A- 0 893 097
- WO-A-97/29698
- WO-A-97/42915
- WO-A-97/47257
- DE-A- 19 628 193
- FR-A- 2 281 095
- US-A- 2 785 673
- US-A- 5 462 548
- US-A- 5 571 111

## Beschreibung

Hüftgelenkprothesen bestehen aus einem Prothesenteil, der den Oberschenkelkopf ersetzt, und einer Pfannenprothese zum Ersatz der Gleitfläche der natürlichen Hüftpfanne. Die Erfindung bezieht sich auf das Einsetzen der Pfannenprothese.

Bevor eine Pfannenprothese in die natürliche Hüftpfanne eingesetzt werden kann, muß letztere passend zu der jeweiligen Pfannenprothese mittels eines Fräserkopfes vorbearbeitet werden. Diese Arbeiten sowie das Einsetzen der Pfannenprothese finden natürlicherweise von der offenen Seite der Hüftpfanne her statt. Die Richtung, in der die Hüftpfanne sich öffnet und die durch den Verlauf ihres Randes bestimmt wird, wird im folgenden als Pfannenachsrichtung bezeichnet. Dies ist in der Regel auch die Richtung, in der der Arzt beim Fräsen der Hüftpfanne die Achsrichtung des Fräsers orientiert und in der er anschließend die Pfannenprothese einbringt. Für diese Arbeiten benötigt der Operateur in herkömmlicher Operationstechnik ein verhältnismäßig weit offenes Operationsfeld. Es muß mindestens so weit sein, daß die Pfannenprothese, der Fräserkopf und ein etwa in Pfannenachsrichtung verlaufendes Instrument eingeführt werden können. Die herkömmliche Operationstechnik verlangt demzufolge, daß das deckende Gewebe in Richtung der Pfannenachse großräumig geöffnet wird.

Es ist ein Instrumentarium bekannt (WO97/47257), das es gestattet, statt einer großen, etwa in Achsrichtung gelegenen Öffnung mit zwei kleinen Operationsöffnungen auszukommen, von denen eine erste, quer zur Pfannenachsrichtung gelegenen Öffnung zum Einführen des Fräserkopfes und der Pfannenprothese dient, also derjenigen Teile, die einen größeren Durchmesser aufweisen und unmittelbar an der Gelenkpfannne benötigt werden. Sie werden im folgenden als Pfannenformteile bezeichnet. Eine zweite, in Achsrichtung gelegene Öffnung dient lediglich zum Einführen der Instrumentenschäfte und kann demzufolge sehr gering sein. Die funktionsnotwendige Verbindung zwischen den Schäften und den Pfannenformteilen geschieht erst, nachdem beide Teile in das Operationsgebiet bei der natürlichen Gelenkpfanne eingeführt wurden. Dank der Verringerung der Operationswunden wird die Beanspruchung des Patienten vermindert.

Bei dem bekannten Gerät ist ein Trag- und Führungsteil für die Instrumente vorgesehen, das an einem Ende eine Führung für je einen Instrumentenschaft bildet, der durch eine Bohrung im Oberschenkelhals zur Hüftpfanne geführt wird. Am anderen Ende des Stützteils ist eine Halterung für Tragarme vorgesehen, an denen die Pfannenformteile befestigt sind. Dies soll helfen, die Schäfte und die zugehörigen Pfannenformteile durch verschiedene Operationsöffnungen in dasselbe Operationsgebiet zu führen und dort achsgleich so zu positionieren, daß sie miteinander gekoppelt werden können. Dies Ziel wird mit' dem bekannten Gerät aber nicht erreicht. Der erfahrene Operateur fühlt sich dadurch eher behindert, weil es ihm nicht gestattet, die Pfannenformteile so zu positionieren, wie es sein anatomisches Gespür verlangt.

Der Erfindung liegt die Aufgabe zugrunde, ein Instrumentarium gemäß dem Oberbegriff des Anspruchs 1 zu schaffen, das dem Operateur größere Zugangsfreiheit gibt und dennoch ein leichtes Kuppeln der Schäfte mit den Pfannenformteilen gestattet. Die erfindungsgemäße Lösung liegt in den kennzeichnenden Merkmalen des Anspruchs 1 und vorzugsweise denjenigen der Unteransprüche.

Jeder Pfannenformteil weist ein Kupplungselement auf, das mit einem komplementären Kupplungselement am Ende des zugehörigen Schafts zu verbinden ist. Erfindungsgemäß ist das Kupplungselement des Pfannenformteils von einer das Schaftende zu ihm hinführenden, trichterförmigen Führungsfläche umgeben. Wenn der Operateur den Pfannenformteil im Operationsgebiet hält und den Schaft zu diesem hinschiebt, trifft er mit dem vorderen Schaftende die Führungsfläche, die es ihm gestattet, die Kupplungselemente der beiden Teile zueinander hinzuführen. Dies fällt ihm leichter, als wenn er gleichzeitig auch noch die Eigenheiten eines die Position der beiden Teile beeinflussenden Tragteils im Auge behalten müßte.

Zweckmäßigerweise ist das Kupplungselement am Pfannenformteil eine Bohrung, die ggf. mit zusätzlichen Verbindungseinrichtungen ausgestattet ist, beispielsweise einem Gewinde oder Bajonettvorsprüngen. Das Kupplungselement des Schafts kann dann von dem zapfenförmigen Ende des Schafts gebildet sein, das in die Bohrung eingesteckt ist und ggf. mit komplementären Verbindungseinrichtungen ausgerüstet ist.

Zum Manipulieren der Pfannenformteile kann ein Griffteil vorgesehen sein, der jeweils mit einem Pfannenformteil lösbar verbunden werden kann. Mittels dieses Griffteils wird der Pfannenformteil eingeführt und so ausgerichtet und gehalten, daß der durch die andere Operationsöffnung eingeführte Schaft damit gekoppelt werden kann. Sobald die Kopplung erfolgt ist, kann der Griffteil gelöst werden, weil die Lage und Ausrichtung des Pfannenformteils nunmehr durch den Schaft bestimmt werden kann. Mittels des Schafts wird der Pfannenformteil betätigt. Darunter wird die Bewegung oder Ausrichtung des Pfannenformteils in bezug auf die natürlichen Gegebenheiten verstanden, beispielsweise die Drehung eines Fräserkopfs zur Bearbeitung der Hüftpfanne oder das Ausrichten und Einpressen einer Pfannenprothese.

Der Kupplungsabschnitt und die Führungsfläche können im Falle eines Fräserkopfes unmittelbar an diesem angeordnet sein. Für Pfannenprothesen und deren Teile ist diese Möglichkeit nicht immer gegeben. Eine ausgedehnte Führungsfläche wird sich daran selten unterbringen lassen. Auch wird es oft nicht möglich oder unerwünscht sein, einen Kupplungsabschnitt vorzusehen. Die Erfindung sieht deshalb einen besonderen Pfannenträgerteil vor, der den Kupplungsabschnitt und ggf. die Führungsfläche trägt und Verbindungseinrichtungen aufweist, die mit der Pfannenprothese zusammenwirken und insbesondere an deren Rand angreifen. Diese Verbindungseinrichtungen brauchen nicht oder nicht vollständig an dem Trägerteil angeordnet zu sein; sie können auch mindestens teilweise von dem Griffteil gebildet sein, der zum Einführen der Pfannenprothese und des Trägerteils dient.

Die Erfindung wird im folgenden näher unter Bezugnahme auf die Zeichnungen erläutert, die ein vorteilhaftes Ausführungsbeispiel veranschaulichen. Darin zeigen:
- Fig. 1: einen schematischen Schnitt durch das Operationsfeld,
- Fig. 2: den Zustand nach Resektion des Hüftkopfs,
- Fig. 3: das Einsetzen einer Acetabulum-Raspel,
- Fig. 4: das Einsetzen einer Hüftpfannenfassung,
- Fig. 5: den Zustand nach dem vorläufigen Positionieren der Hüftpfannenfassung,
- Fig. 6: eine alternative Instrumentenausführung zu Fig. 4 und 5,
- Fig. 7: die Verbindung des Instruments gemäß Fig. 4 mit einem Griff,
- Fig. 8: eine Seitenansicht der Teile gemäß Fig. 7,
- Fig. 9: das Einsetzen eines Einschlaginstruments für die Pfannenfassung,
- Fig. 10: das Einsetzen einer Pfannenauskleidung.

Der Beckenknochen 1 bildet bekanntlich die Hüftpfanne 2, das sogenannte Acetabulum, mit einer Gelenkfläche für den Hüftkopf 3, der über den Schenkelhals 4 das proximale Ende des Oberschenkelknochens 5 bildet. Zwischen der Körperoberfläche (Haut) 6 und dem Gelenk befindet sich deckendes Gewebe 7. Die Ränder 8 der natürlichen Hüftpfanne 2 bilden eine Öffnung, die eine Öffnungsrichtung erkennen läßt, die als Pfannenachsrichtung durch die strichpunktierte Linie 9 angegeben ist. Etwa dieselbe Richtung hat der Schenkelhals 4 in Standstellung.

Wenn der Hüftkopf 3 und die Oberfläche der Hüftpfanne 2 durch Prothesenteile ersetzt werden sollen, wird der Hüftkopf 3 durch einen in Richtung der strichpunktierten Linie 10 verlaufenden ersten Schnitt 25 reseziert und entfernt, der im wesentlichen lotrecht zur Richtung 9 verläuft.

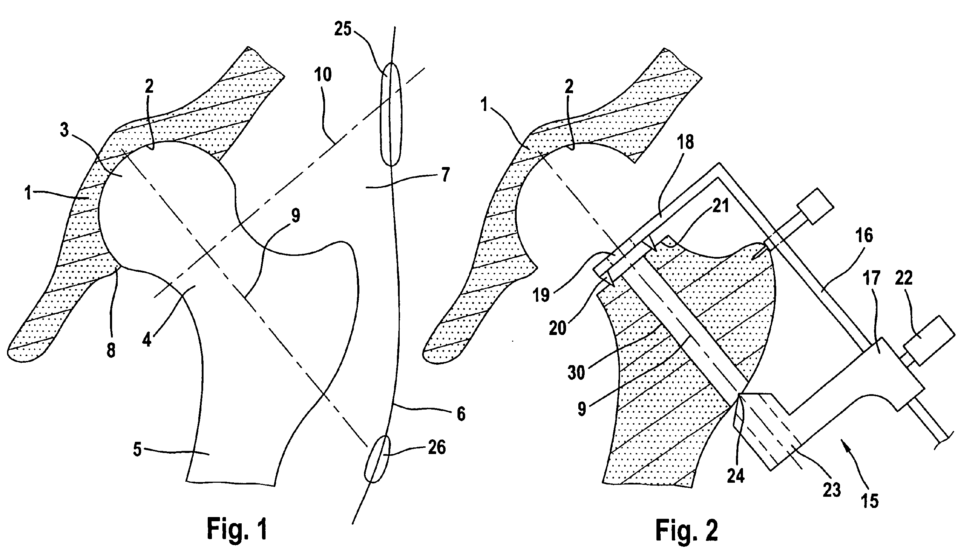

Um Zugang für die oben erwähnten Insrumentenschäfte zu bieten, wird ein zweiter Schnitt in der Richtung 9 geführtund anschließend ein Zielgerät 15 an den Oberschenkelknochen etwa in der in Fig. 2 veranschaulichten Weise angesetzt. Dieses Gerät besteht aus einem Bügel 16 und einem Stellschlitten 17. Der Bügel 16 weist an seinem Ende in einem abgewinkelten Teil 18 eine erste Zielbohrung 19 auf. Außerdem können Spitzen 20 vorgesehen sein, die für einen sicheren Sitz an der Resektionsfläche 21 des Knochens sorgen. Am Stellschlitten 17, der in Längsrichtung des Bügels 16 verschiebbar und mittels einer Schraube 22 in der gewünschten Stellung fixiert werden kann, befindet sich eine zweite Zielbohrung 23, die mit der Bohrung 19 fluchtet. Auch am Stellschlitten 17 können Spitzen 24 zur besseren Fixierung des Gerätes am Knochen vorgesehen sein.

Das Gerät ist in der gezeigten Form als Bohrlehre ausgebildet. Statt dessen oder zusätzlich kann es auch zum Einbringen eines Führungsdrahtes für einen Hohlbohrer ausgebildet sein. Diese Technik ist bekannt.

Mit Hilfe des Gerätes 15 wird in den Oberschenkelknochen eine Bohrung 30 gebohrt, die bei entspannter Beinlage im wesentlichen der Pfannenachsrichtung 9 entspricht. Die Bohrung 30 beginnt im intertrochantären Bereich und sollte etwa mittig in der Resektionsfläche 21 münden, damit später davon ausgehend im Oberschenkelknochen die Aufnahmehöhlung für den Oberschenkel-Prothesenteil geformt werden kann. Durch diese Bohrung finden die Instrumentenschäfte etwa koaxialen Zugang zur Hüftpfanne 2.

Der nächste Schritt betrifft die Vorbereitung der Hüftpfanne 2 zur Aufnahme der Pfannnenprothese. Dies geschieht mittels eines Fräserkopfes 35, der aus einer herkömmlich ausgebildeten Fräserkopfschale 36 und einem Trägerteil 37 besteht, der fest mit dem Rand der Fräserschale 36 verbunden ist und mittig eine Bohrung 38 enthält, die mit radialen Bajonettvorsprüngen 39 versehen ist. Sie bildet das fräserseitige Kupplungselement.

Am Ende des Schaftes 40 sind innerhalb eines zur Bohrung 38 passenden Abschnittes 41 Bajonettnuten 42 passend zu den Vorsprüngen 39 vorgesehen. Wenn man das Ende des Schaftes 40 in die Bohrung 38 einführt und in der Richtung der Fräsbewegung dreht, kommen die Kupplungsabschnitte miteinander in Eingriff. Das Einfügen des Abschnitts 41 in die Bohrung 38 wird dadurch erleichtert, daß die Bohrung 38 von einer Trichterfläche 43 umgeben ist, die zu ihr hinführt.

Um den Fräserkopf 36 zu halten, während er zur Hüftpfanne geführt und dort mit dem Schaft verbunden wird, ist er mit einem Griffteil 45 verbunden, der leicht gelöst werden kann, sobald der Fräserkopf 35 und der Schaft 40 miteinander gekuppelt sind.

Der Operateur kann die Fräsrichtung weitgehend beliebig einstellen, indem entweder die Bohrung 30 im Oberschenkelknochen so geräumig gemacht wird, daß sie Schwenkbewegungen und Bewegungen quer zur Schaftachse zuläßt, oder indem das Bein des Patienten entsprechend gelagert wird.

Nachdem die Hüftpfanne 2 ausgefräst ist, wird der Fräskopf 35 mittels des Schaftes 40 aus der Hüftpfanne herausgehoben. Anschließend wird der Fräserkopf vom Schaft gelöst und kann durch die erste Öffnung 25 wieder entnommen werden. Dafür kann wieder der Griffteil 45 oder irgendein anderes Instrument eingesetzt werden.

In manchen Fällen wünscht man die Resektionsfläche 21 noch nachzubearbeiten (DE-A-3216533). Man verwendet dann einen nicht dargestellten Fräserkopf, dessen Fräseroberfläche der Resektionsfläche 21 zugewendet ist und ebenfalls eine Kupplungsbohrung 38 zur Verbindung mit dem Schaft 40 aufweist. Die Führung des Schaftes 40 in der Bohrung 30 gewährleistet dann, daß die nachbearbeitete Resektionsfläche 21 die gewünschte Ausrichtung gegenüber der Bohrung 30 hat. Wenn diese Bohrung dank dem Zielgerät 15 dieselbe Richtung gegenüber dem Knochen hat, in der später der Schaft des oberschenkelseitigen Prothesenteils aus der Resektionsfläche 21 austreten soll, ist dadurch auch eine korrekte Ausrichtung der Resektionsfläche im Verhältnis zu diesem Prothesenteil gesichert.

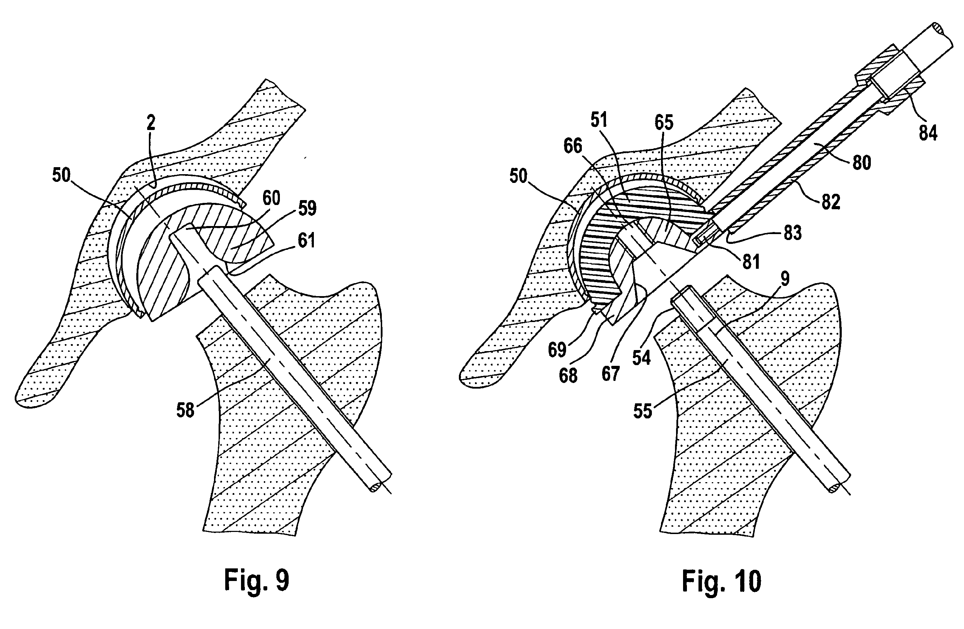

Nachdem die Fräsarbeiten abgeschlossen sind, wird durch die Operationsöffnung 25 mittels des Griffteils 45 die Pfannenfassung 50 eingeführt. Im dargestellten Beispiel handelt es sich um eine Prothesenschale, die in der Hüftpfanne 2 zu verankern ist und die ihrerseits noch eine Pfannenauskleidung 51 (siehe Fig. 10) beispielsweise aus Polyethylen aufnehmen wird.

Mit der Pfannenfassung 50 ist ein Trägerteil 52 in später erläuterter Weise lösbar verbunden, der ebenso wie der Halter 37 des Fräserkopfes 35 eine trichterförmige Führungsfläche 43 und eine Bohrung 53 als Kupplungselement aufweist, die mit einem Gewinde versehen ist zur Verbindung mit dem ein entsprechendes Gewinde tragenden Kupplungselement 54 am Ende des Schaftes 55. Wenn der Trägerteil 52 und die Pfannenfassung 50 mit dem Schaft 55 verbunden sind, kann der Griff 45 gelöst werden. Jedoch ist dies nicht unbedingt erforderlich, sofern der Griff nicht an der Pfannenfassung 50 sondern an dem Trägerteil 52 angreift.

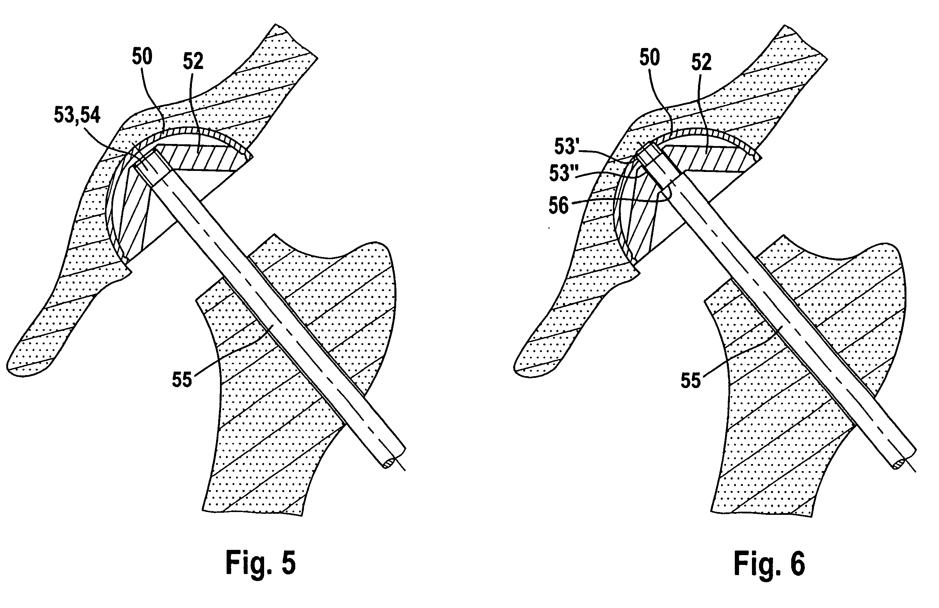

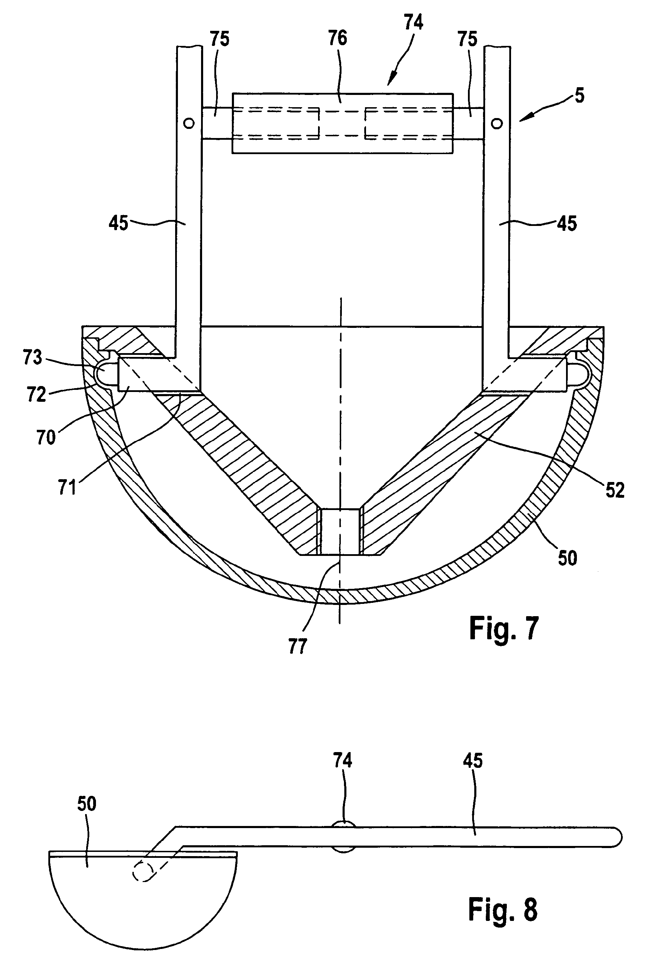

Mittels des Schaftes 55 wird nun die Pfannenfassung 50 in der gewünschten Richtung in das Acetabulum 2 gestoßen (Fig. 5). Danach wird der Pfannenträgerteil 52 von der Pfannenfassung 50 gelöst und mittels des Schaftes 55 aus der Pfannenfassung 50 herausgehoben. Er kann dann vom Schaft 55 gelöst und mittels des Griffs 45 oder eines anderen geeigneten Instrumentes durch die Operationsöffnung 25 aus dem Operationsfeld entfernt werden. Fig. 7 veranschaulicht ein Ausführungsbeispiel des Griffteils 5 und seines Zusammenwirkens mit einer Pfannenfassung 50 und dem zugehörigen. Trägerteil 52 in vergrößertem Maßstab. Der Griffteil 5 ist ein U-förmig gebogener Bügel, dessen Schenkel 45 in entgegengesetzt nach außen weisenden Haken 70 enden. Der Tägerteil 52 weist zwei diametral angeordnete, fluchtende Bohrungen 71 auf, durch die die Haken 70 im montierten Zustand des Griffteils 5 hindurchgreifen. Die Pfannenfassung 50 enthält eine umlaufende Nut 72, die beispielsweise zur späteren Aufnahme eines Rastelementes des Pfanneneinsatzes 51 bestimmt ist. An den Enden der Haken 70 befinden sich vorragende Spitzen 73, die in der dargestellten, montierten Stellung des Griffteils 5 in die Nut 72 eingreifen. Dadurch ist nicht nur der Griffteil 5 mit dem Trägerteil 52 sondern dieser auch mit der Pfannenfassung 50 fest verbunden.

Die Schenkel 45 des Bügels 5 sind miteinander durch eine Spreizeinrichtung 74 verbunden, die aus zwei mit gegensinnigem Gewinde versehenen Gewindezapfen 75 und einer dazu passenden Gewindehülse 76 besteht. Dreht man die Gewindehülse 76 in einer Richtung, so werden die Schenkel gespreizt. Dreht man sie in der anderen Richtung, so werden die Schenkel zusammengezogen. Fig. 7 zeigt die Schenkel im gespreizten Zustand, in welchem der Griffteil mit dem Trägerteil 52 und der Pfannenfassung 50 fest verbunden ist. Will man den Trägerteil 52 aus der Pfannenfassung 50 lösen, so zieht man die Schenkel 45 des Griffteils 5 durch Drehung der Gewindehülse ein wenig zusammen, so daß die Spitzen 73 sich aus der Nut 72 lösen, wobei die Haken 70 sich aber noch in den Bohrungen 71 des Trägerteils befinden. Dieser kann nun mit dem Griffteil abgenommen werden. Bei weiterem Zusammenziehen der Griffschenkel 45 kann der Griff auch von dem Trägerteil abgenommen werden. Das Umgekehrte vollzieht sich beim Zusammensetzen dieser Teile.

Einfachheitshalber ist der Griffteil 5 in Fig. 7 so gezeichnet, als erstrecke er sich etwa in der Richtung der Achse 77. In Wirklichkeit erstreckt er sich aber quer dazu, wie man Fig. 8 entnehmen kann.

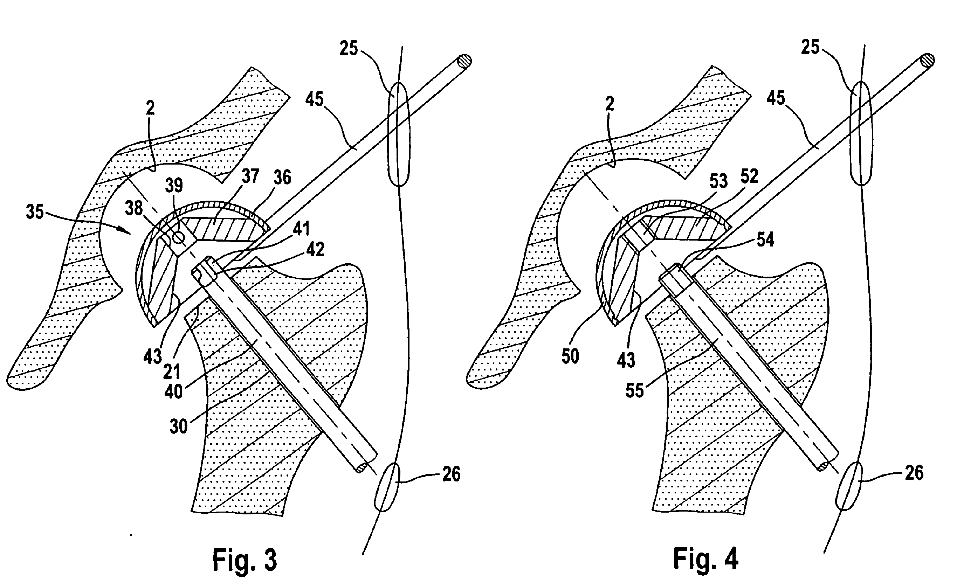

Während bei der Ausführung gemäß Fig. 4 und 5 das Kupplungselement 54 ausschließlich an dem Trägerteil 52 angreift, ist in Fig. 6 eine Alternativausführung dargestellt, die insbesondere dann benutzt wird, wenn die Pfannenfassung 50 am Boden mit einer Gewindebohrung 53' versehen ist. Der Trägerteil 52 weist dann statt der Gewindebohrung 53 lediglich eine Durchgangsbohrung 53" auf. Der Schaft 55 setzt sich mit einem Bund 56 auf die die Bohrung 53" umgebende Fläche auf und überträgt an dieser Stelle die zum Einstoßen der Pfannenfassung 50 in das Acetabulum erforderliche Kraft.

Wenn der Arzt Zweifel hat, ob mit dem in Fig. 4 bis 8 gezeigten Instrument schon ein hinreichend fester Sitz der Prothesenschale 50 in der Hüftpfanne 2 erzielt ist, kann er mittels des in Fig. 9 gezeigten Instrumentes nachstoßen. Das Instrument besteht aus einem Schaft 58 und einem Stößelkopf 59, der an einem nicht gezeigten Griff angeordnet ist und eine Aufnahmebohrung 60 für das zylindrische Ende des Schaftes 58 aufweist. Die Bohrung 60 ist von einer Führungsfläche 61 umgeben, die in diesem Beispiel eine etwas andere Trichterform aufweist als die in Fig. 3 und 4 gezeigte. Sie schließt mit ihrer Mittellinie (Pfannenachsrichtung 9) einen zur Mitte hin kleiner werdenden Winkel ein, so daß der Führungseffekt um so stärker wird, je mehr sich das Ende des Schaftes 58 der Bohrung 60 nähert. Der Stößelkopf 59 hat eine sphärische Außenform passend zur Innenform der Pfannenfassung 50. Er kann sie daher ohne Gefahr einer Deformation in das Acetabulum 2 eintreiben. Danach kann der Schaft 58 aus dem Stößelkopf 59 einfach herausgezogen und dieser aus dem Operationsgebiet entfernt werden.

In entsprechender Weise kann gemäß Fig. 10 der Pfanneneinsatz 51 in die Pfannenfassung 50 eingesetzt werden. Der Einsatz 51 ist beispielsweise mit einer Rasteinrichtung ausgerüstet, die ihn in der Pfannenfassung festhält (Beispiel: WO 99 60 955). Zur Montage des Einsatzes ist es daher nur erforderlich, ihn in der Pfannenachsrichtung in die Pfannenfassung einzustoßen.

Dies gelingt mittels eines Schafts 55, der am vorderen Ende (wie in Zusammenhang mit Fig. 4 erläutert) einen Gewindeabschnitt 54 aufweist und mit einem Stößelkopf 65 verbindbar ist, der eine entsprechende Gewindebohrung 66 und eine diese umgebende Führungsfläche 67 aufweist. Der Stößelkopf 65 hat einen vorstehenden Kragen 68, der mit dem Rand des Pfanneneinsatzes 51 zusammenwirken kann, um ihn genau auf die Pfannenrichtung einzustellen. Dies ist nicht unbedingt erforderlich, wenn der Einsatz selbst auch mit einem vorstehenden Kragen 69 versehen ist, der sich am Rand der Prothesenschale 50 anlegt und dadurch an dieser ausgerichtet wird.

Der Stößelkopf 65 ist mit einer Griffstange 80 verbunden. Er ist beispielsweise mit dieser bei 81 lösbar verschraubt. Um den Pfanneneinsatz 51 an dem Stößelkopf festzuhalten, bevor er sicher in der Pfannenfassung 50 verankert ist, ist die Griffstange 80 von einem Fixierrohr 82 umgeben, das am stößelseitigen Ende eine Kante 83 aufweist und über ein Gewinde 84 mit der Griffstange 80 verbunden ist. Durch Verdrehung des Fixierrohrs kann es gegenüber der Griffstange vor- und zurückgeschraubt werden, um in der einen Endstellung mittels der Kante 83 den Pfanneneinsatz 51 am Stößelkopf 65 festzuhalten oder in einer zurückgezogenen Stellung sie freizugeben.

Nachdem der Einsatz 51 in der Schale 50 eingeschnappt ist, kann der Stößelkopf 65 mit dem Schaft 55 zurückgezogen, von diesem gelöst und aus dem Operationsfeld durch die Operationsöffnung 25 entfernt werden.

## Patentansprüche

1. Instrumentarium zum Einsetzen einer Hüftpfannenprothese, mit mindestens einem Pfannenformteil (35, 50, 52, 59, 65) und einem damit axial zu verbindenden Schaft (40, 55, 58), der am Ende ein Kupplungselement (41, 54) aufweist, das mit einem entsprechenden Kupplungselement (38, 53, 60, 66) am Pfannenformteil (35, 50, 52, 59, 65) zu verbinden ist, sowie mit einem quer zur Pfannenachsrichtung (9) mit dem Pfannenformteil (35, 50, 52, 59, 65) lösbar verbindbaren Griffteil (45), **dadurch gekennzeichnet, daß** das Kupplungselement (38, 53, 60, 66) am Pfannenformteil (35, 50, 52, 59, 65) von einer das Schaftende zu ihm hinführenden, trichterförmigen Führungsfläche (43, 61, 67) umgeben ist.

2. Instrumentarium nach Anspruch 1, **dadurch gekennzeichnet, daß** das Kupplungselement am Pfannenformteil (35, 50, 52, 59, 65) eine Bohrung (38, 53, 60, 66) ist.

3. Instrumentarium nach Anspruch 1 oder 2, **dadurch gekennzeichnet, daß** der Pfannenformteil ein Teil einer Pfannenprothese (50) ist und die Führungsfläche an einem Pfannenträgerteil (52) angeordnet ist, der mit dem Pfannenformteil (50) zusammenwirkende Verbindungseinrichtungen (72, 73) aufweist und mit dem Griffteil verbunden oder verbindbar ist.

4. Instrumentarium nach Anspruch 3, **dadurch gekennzeichnet, daß** die Verbindungseinrichtungen (72) auf seiten des Pfannenformteils am Rand der Pfannenprothese (50) vorgesehen sind.

5. Instrumentarium nach Anspruch 3 oder 4, **dadurch gekennzeichnet, daß** die Verbindungseinrichtungen (72, 73) mindestens teilweise von dem Griffteil (45) gebildet sind.

## Claims

1. An instrument set for inserting an acetabular prosthesis with at least one shaped acetabular part (35, 50, 52, 59, 65) and a shaft (40, 55, 58) which is to be connected axially thereto and which at one end has a coupling element (41, 54) which is to be connected to a corresponding coupling element (38, 53, 60, 66) on the shaped acetabular part (35, 50, 52, 59, 65), and with a grip part (45) which can be releasably connected to the shaped acetabular part (35, 50, 52, 59, 65) transverse to the axial direction (9) of the acetabulum, in which the coupling element (38, 53, 60, 66) on the shaped acetabular part (35, 50, 52, 59, 65) is surrounded by a funnel-shaped guide surface (43, 61, 67) guiding the shaft end toward it.

2. The instrument set as claimed in claim 1, in which the coupling element on the shaped acetabular part (35, 50, 52, 59, 65) is a bore (38, 53, 60, 66).

3. The instrument set as claimed in claim 1 or 2, in which the shaped acetabular part is part of a prosthetic socket (50) and the guide surface is arranged on a socket support part (52) which has connection devices (72, 73) cooperating with the shaped acetabular part (50) and which is or can be connected to the grip part.

4. The instrument set as claimed in claim 3, in which the connection devices (72) on the side of the shaped acetabular part are provided on the edge of the prosthetic socket (50).

5. The instrument set as claimed in claim 3 or 4, in which the connection devices (72, 73) are formed at least partially by the grip part (45).

## Revendications

1. Ensemble d'instruments destiné à insérer une prothèse de l'acetabulum, qui présente au moins une pièce façonnée d'acetabulum (35, 50, 52, 59, 65) et une tige (40, 55, 58) destinée à y être reliée axialement et qui présente à son extrémité un élément d'accouplement (41, 54) destiné à être relié à un élément d'accouplement correspondant (38, 53, 60, 66) prévu sur la pièce façonnée d'acetabulum (35, 50, 52, 59, 65), ainsi qu'une pièce de saisie (45) qui peut être reliée de manière libérable à la pièce façonnée d'acetabulum (35, 50, 52, 59, 65), transversalement par rapport à la direction de l'axe (9) de l'acetabulum, **caractérisé en ce que** l'élément d'accouplement (38, 53, 60, 66) prévu sur la pièce façonnée d'acetabulum (35, 50, 52, 59, 65) est entouré par une surface de guidage (43, 61, 67) en forme d'entonnoir qui guide l'extrémité de la tige vers lui.

2. Ensemble d'instruments selon la revendication 1, **caractérisé en ce que** l'élément d'accouplement prévu sur la pièce façonnée d'acetabulum (35, 50, 52, 59, 65) est un alésage (38, 53, 60, 66).

3. Ensemble d'instruments selon la revendication 1 ou 2, **caractérisé en ce que** la pièce façonnée d'acetabulum fait partie d'une prothèse d'acetabulum (50) et **en ce que** la surface de guidage est disposée sur une pièce (52) de support d'acetabulum qui présente des dispositifs (72, 73) de liaison qui coopèrent avec la pièce façonnée d'acetabulum (50) et qui est reliée ou peut être reliée à la pièce de saisie.

4. Ensemble d'instruments selon la revendication 3, **caractérisé en ce que** les dispositifs de liaison (72) sont prévus du côté de la pièce façonnée d'acetabulum sur le bord de la prothèse d'acetabulum (50).

5. Ensemble d'instruments selon la revendication 3 ou 4, **caractérisé en ce que** les dispositifs de liaison (72, 73) sont formés au moins en partie par la pièce de saisie (45).
